# EUROPEAN PATENT APPLICATION

(11) **EP 4 714 346 A1**
(43) Date of publication of application: **25.03.2026**
(21) Application number: 25200774.5
(22) Date of filing: 08.09.2025
(51) Int. Cl.: A61B 5/11, A61B 5/00, A61B 7/00

(54) **JAW HEALTH METRIC USING HEADPHONES**

(30) Priority: 19.09.2024 US 202463696754 P; 26.08.2025 US 202519310090
(71) Applicant: Apple Inc., Cupertino, CA 95014 (US)
(72) Inventor: MURGAI, Prateek, Cupertino, 95014 (US)
(74) Representative: Barton, Russell Glen

(57) **Abstract**

Jaw health systems and methods are described in which headphone devices are used to generate metrics related to the jaw health of a user. In one aspect, headphone devices worn on either side of the jaw may each determine the number of bites and chews of a user during an ingestion process and may then generate an imbalance metric. In one aspect, the headphone devices may be used to determine a food or substance toughness during the ingestion process.

## Description

### FIELD

Aspects described herein relate to chewing-related health metrics for jaw health.

### BACKGROUND INFORMATION

Mastication or chewing is the process of physically breaking down food for digestion. During this process, the muscles of mastication (e.g., masseter, temporalis, medial pterygoid, lateral pterygoid, etc.) as well as the mandibular nerves facilitate chewing by elevating the lower jaw or mandible to repeatedly bring the teeth of a user into intermittent contact. Further, the temporomandibular joint, which connects the lower jaw or mandible to the temporal bone of the skull, may experience significant pressure during mastication or chewing.

### SUMMARY

Jaw health systems, methods and devices are described. In an aspect, a jaw health system may include a first headphone device and a second headphone device that include a first set of sensors and a second set of sensors, respectively, to generate first-side user data (e.g., left side) and second-side user data (e.g., right side) related to an ingestion process, where the ingestion process includes at least a first bite to sever a portion of food and a first chew to further break down the portion of food with the user's teeth. In addition, a processor of the jaw health system may receive and analyze the first-side user data and the second-side user data to determine a first number of chews detected by the first headphone device and a second number of chews detected by the second headphone device, respectively. Further, the jaw health system may then generate an imbalance metric based on the comparison of the first number of chews detected by the first headphone device and the second number of chews detected by the second headphone device. In another aspect, the jaw health system may also analyze the toughness of a portion of food chewed during the ingestion process and then generate a toughness metric based on a toughness threshold.

### BRIEF DESCRIPTION OF THE DRAWINGS

FIG. 1 shows example headphone devices and a companion device in accordance with an aspect.
FIG. 2A is a schematic illustration of example headphone devices and a companion device in accordance with an aspect.
FIG. 2B is a schematic illustration of example headphone devices and a companion device in accordance with another aspect.
FIG. 3 is a schematic illustration of a training phase in accordance with an aspect.
FIG. 4 is a flow chart of a method for generating a jaw health metric in accordance with an aspect.

### DETAILED DESCRIPTION

It has been observed that chewing is a semi-autonomic act where a user may fall into particular habits with little to no awareness of the formation of such habits. For example, over-chewing on one side of the mouth while under-chewing on the other side may not be noticeable to the user during the ingestion process. However, over time it has been observed that such habits can lead to pain in the jaw, such as temporomandibular joint dysfunction (TMJD). Further, it has been observed that parafunctional habits not related to chewing (e.g., teeth grinding, jaw clenching, etc.) may also be semi-autonomic and lead to similar pain or discomfort in the teeth, lower jaw or temporomandibular joint. In accordance with various aspects of the disclosure here, a jaw health system determines a jaw health metric based on user data (e.g., first-side user data, second-side user data) generated by the sensors of a headphone device (e.g., earbuds). In one aspect, the sensors of the headphone device may generate user data related to the start and stop of a bite as well as the chew frequency within each bite, where such data may then be analyzed to determine an imbalance metric during the ingestion process (e.g., over-chewing on the left side, over-chewing on the right side, etc.). In another aspect, the sensors of the headphone device may generate user data related to the hardness or toughness of the food or substance being bitten and/or chewed by the user, where such data may then be analyzed to determine a toughness metric during the ingestion process (e.g., soft, hard, etc.). In another aspect, the jaw health system may provide notifications to the user related to such individual metric and/or an overall jaw health metric and may then provide predictions related to such metrics. In this way, the jaw health system may bring about an awareness of the user's chewing habits (or other parafunctional habits not related to chewing) so that the user may track their jaw health and potentially take steps to eliminate or at least curtail such activity to attain better overall jaw health.

The various aspects are described with reference to figures. However, certain aspects may be practiced without one or more of these specific details, or in combination with other known methods and configurations. In the following description, numerous specific details are set forth, such as specific configurations, dimensions and processes, etc., in order to provide a thorough understanding of the aspects. In other instances, well-known digital audio signal processing techniques processes have not been described in particular detail in order to not unnecessarily obscure the aspects. Reference throughout this specification to "one aspect" means that a particular feature, structure, configuration, or characteristic described in connection with the aspect is included in at least one aspect. Thus, the appearances of the phrase "in one aspect" in various places throughout this specification are not necessarily referring to the same aspect. Furthermore, the particular features, structures, configurations, or characteristics may be combined in any suitable manner in one or more aspects.

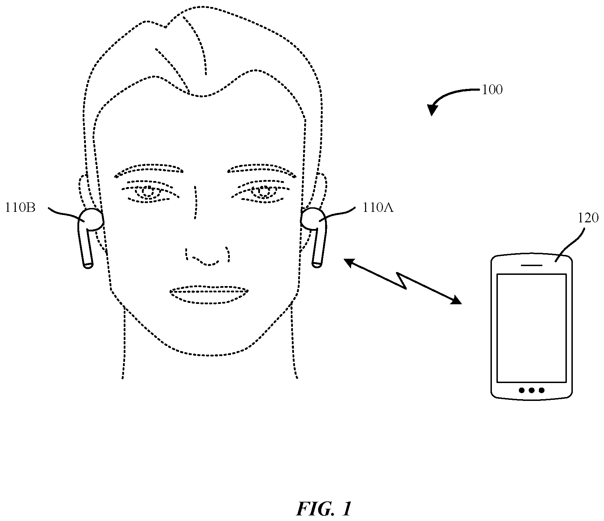

Referring now to FIG. 1, example headphone devices and a companion device are shown in accordance with an aspect. Jaw health system 100 may include one or more headphone devices 110 (e.g., headphone device 110A, headphone device 110B, etc.) and companion device 120. The one or more headphone devices 110 may include any type of earbuds or in-ear headphones, on-the-ear headphones, over-the-ear headphones, etc., where the aspects here may work in various types of headphones. Further, the one or more headphone devices 110 may be implemented as part of jaw health system 100 either individually (e.g., one ear) or as a pair (e.g., both ears). In an aspect, jaw health system 100 may include a pair of headphone devices to be worn on either side of the jaw of the user (e.g., left earbud and right earbud) so that user data (e.g., first-side user data, second-side user data) may be received by and shared between both devices to build better confidence in the dataset. In the example of FIG. 1, jaw health system 100 may include first headphone devices 110A and second headphone device 110B that may be in communication with companion device 120, where companion device 120 may include a mobile device, tablet, laptop, or any other suitable computing device. For example, companion device 120, such as a smartphone, may be paired for communication with the headphone devices via a wireless communication link, such as a BLUETOOTH link. Further, the operations of jaw health system 100 may be performed and implemented in whole or in part on headphone devices 110A, 110B and/or companion device 120.

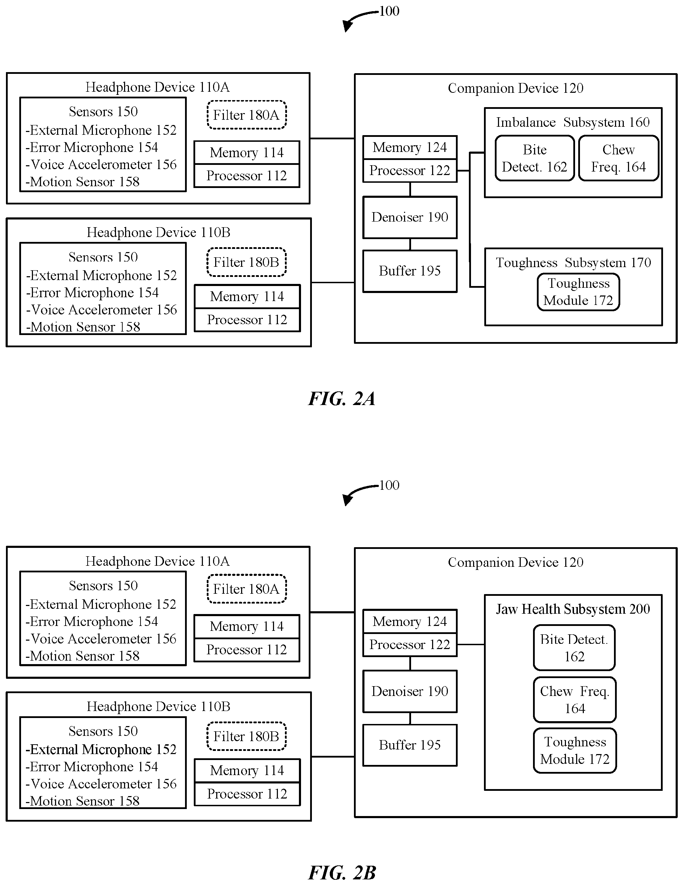

Referring now to FIG. 2A, a schematic illustration of example headphone devices and a companion device are shown in accordance with aspects. The headphone devices and companion devices may include a processor (e.g., central processing unit, processing circuitry, etc.), memory (e.g., read-only memory, random access memory, etc.), sensors (e.g., external microphones, error microphones, voice accelerometers, motion accelerometers, motion sensors, gyroscopes, magnetometers, etc.) and power supplies contained within a housing. Further, the processor may also include networking technology to enable wireless communication (e.g., Bluetooth, Wi-Fi, etc.) between the headphone devices and companion device. In the example of FIG. 2A, headphone devices 110A, 110B may each include processor 112 coupled to memory 114, where processor 112 may execute a set of instructions stored on memory 114 to receive user data (e.g., first-side user data, second-side user data) generated by sensors 150, where such components may be contained within a housing. Further, companion device 120 may include processor 122 coupled to memory 124, where processor 122 may execute a set of instructions stored on memory 124 to receive and perform operations on the user data (e.g., first-side user data, second-side user data) generated by headphone devices 110A, 110B.

In further reference to FIG. 2A, a processor of jaw health system 100 (e.g., processor 112, processor 122, etc.) may cause audio and motion analyses to be performed on the user data generated by the sensors of the headphone devices, which may include but are not limited to filtering signals, removing noise, adding annotations/labels, converting signals into corresponding visual representations (e.g., waveforms, spectrum plots, spectrograms, etc.), etc. Such analyses can include one or more learning-based and/or non-learning-based systems for perceiving, synthesizing, and inferring information. For example, persons skilled in the art will appreciate that imbalance subsystem 160 can include any suitable number of processes (e.g., bite detection module 162, chew frequency module 164, etc.) to generate an imbalance metric based on input from sensors 150, where such processes may be performed by learning-based and/or non-learning-based systems. Similarly, persons skilled in the art will appreciate that toughness subsystem 170 can include any suitable number of processes (e.g., toughness module 172, etc.) to generate a toughness metric based on input from sensors 150, where such processes may be performed by learning-based and/or non-learning-based systems.

In instances where jaw health system 100 is a learning-based system (e.g., imbalance subsystem 160 and/or toughness subsystem 170), persons of ordinary skill in the art will appreciate that jaw health system 100 can include any suitable machine learning models that are well-known or widely available such as regression techniques, classification techniques, neural networks, and deep learning networks. For instance, jaw health system can include neural networks such as Artificial Neural Network (ANN), Convolutional Neural Network (CNN), Recurrent Neural Network (RNN), Adversarial Network (GAN), Reinforcement Learning Model (RLM), Encoder/Decoder Networks, and/or Transformer-Based Models (e.g., Bidirectional Encoder Representations from Transformers (BERT), Generative Pre-trained Transformer (GPT), and/or a multi-modal large language model (LLM)). Additionally, or alternatively, persons of ordinary skill in the art will appreciate that jaw health system 100 (e.g., imbalance subsystem 160 and/or toughness subsystem 170) can be any suitable non-learning processes such as rule-based systems, heuristics, decision trees, knowledge-based systems, statistical or stochastic systems, and expert systems. In instances where jaw health system 100 is a non-learning-based system, imbalance subsystem 160 and/or toughness subsystem 170 can include a pre-defined set of rules or a pre-defined structure to make decisions based on the inputs that the process sees. In one example, non-learning processes such as imbalance subsystem 160 and/or toughness subsystem 170 may determine health of the jaw based on a set of rules conditioned on inputs from weighting sensors 150, a mode of operation of the headphone device, an activity threshold of the user, a playback volume threshold of the headphone devices, and/or an ambient noise threshold of the headphone devices, etc. In such instances, as well as other instances described below, imbalance subsystem 160 and/or toughness subsystem 170 may incorporate learning-based models, non-learning-based models, or any combination thereof.

In further reference to FIG. 2A, headphone devices 110A, 110B may each include sensors 150, where such sensors may include individual sensors (e.g., external microphone, error microphone, voice accelerometer, motion accelerometer, gyroscope, magnetometer, etc.) and/or a sensor package that may include multiple sensors combined in a single unit, such as an inertial measurement unit (IMU) that may include an accelerometer (e.g., motion accelerometer, voice accelerometer, etc.), gyroscope, magnetometer, etc. In one example, sensors 150 may include external microphone 152, error microphone 154, and one or more other sensors whose output signals are interpreted by the processor 112 as bone conduction pickup of the wearer's voice and detected jaw/head movements related to biting or chewing, where such sensors are depicted in FIG. 2A as a voice accelerometer 156 and a motion sensor 158 (e.g., IMU, etc.), respectively. External microphone 152 (e.g., reference microphone) may be located on or near an exterior of headphone devices 110A, 110B to directly detect ambient noise or sound in an environment. Error microphone 154 may be located on an interior of headphone devices 110A, 110B to detect that part of the ambient noise which passes through the sound insulating properties of headphone devices 110A, 110B and into the ear of the user. Voice accelerometer 156 may detect vibrations propagated through tissue and bone of the user, where such vibrations may include voiced speech (e.g., vibrations generated by the vocal cords of the user, etc.) as well as unvoiced speech (e.g., vibrations generated without the use of the vocal cords of the user, etc.). Motion sensor 158 (e.g., IMU, etc.) may detect jaw and/or head movements related to biting or chewing, or other movements that may affect the receipt of data generated by sensors 150. It has been observed that the act of biting and chewing particularly "lights up" both the error microphone and voice accelerometer of a headphone device (such as headphone devices 110A, 110B) so that the error microphone and voice accelerometer may be most heavily weighted in performing the operations of jaw health system 100. However, the weighting of user data (e.g., first-side user data, second-side user data) generated by sensors 150 may be affected by several other factors.

In some aspects, the weighting of user data generated by sensors 150 may be performed as a non-learning-based or ruled-based operation. In one example, the weighting of user data generated by sensors 150 may be based on a mode of operation (e.g., active noise cancellation ("ANC") mode, transparency mode, adaptive audio mode, off mode, etc.). For instance, a processor of jaw health system 100 (e.g., processor 112, processor 122, etc.) may determine that headphone devices 110A, 110B are in ANC mode (e.g., external noise canceled) and may cause user data generated by error microphone 154 to be weighted more, as opposed to when headphone devices 110A, 110B are in transparency mode (e.g., external noise permitted) which may cause user data generated by error microphone 154 to be weighted less. In another example, the weighting of user data generated by sensors 150 may be based on an activity of the user (e.g., walking, etc.), where such an activity may introduce noise into the user data generated by sensors 150. For instance, based on data generated by motion sensor 158, a processor of jaw health system 100 (e.g., processor 112, processor 122, etc.) may determine that an activity level exceeds an activity threshold and may cause user data generated by the voice accelerometer, for example, to be weighed less during the determined activity. In another example, the weighting of user data generated by sensors 150 may be based on a playback volume produced by a speaker of headphone devices 110A, 110B. For instance, based on user data generated by error microphone 154 and voice accelerometer 156, a processor of jaw health system 100 (e.g., processor 112, processor 122, etc.) may determine that the playback volume and its residual echo exceeds a playback threshold and may cause user data generated by external microphone 152 to be weighted more. In another example, the weighting of user data generated by sensors 150 may be based on a level of ambient noise in an environment of the user. In such instances, a processor of jaw health system 100 (e.g., processor 112, processor 122, etc.) may determine that the level of ambient noise in an environment exceeds an ambient noise threshold (e.g., ambient sound pressure level, etc.) and may cause user data generated by external microphone 152 to be weighed less.

In other aspects, the weighting of user data generated by sensors 150 may be performed, at least in part, by a learning-based or machine learning model. The machine learning model may be implemented by suitably programming one or more of the processors/processing circuitries in the system (e.g., by processor 112 being configured according to instructions stored in the memory 114.) For example, the machine learning models of jaw health system 100 (e.g., imbalance subsystem 160 and/or toughness subsystem 170) may include a machine learning encoder-decoder to analyze the detected inputs from sensors 150 to produce a set of context vectors, which may then be used by the decoder to generate an image (e.g., spectrogram) that corresponds to the sensed inputs, where the generated images may then be further analyzed. In some instances, the weighting of sensors 150 may be performed by the machine learning model, such as when spectrogram data generated by motion sensor 158 exceeds a threshold, for example, that may cause the spectrogram data generated by voice accelerometer 156 to be weighed less, similar to the examples described above. It should also be noted that the weighting of sensors 150 may also be performed by a combination of non-learning-based and learning-based systems where, for example, the weighting of the sensors for the mode of operation may be performed by a non-learning-based system and the weighting of the sensors for an activity threshold of the user may be performed by a learning-based system. Further, such examples are merely illustrative, not exhaustive, where other combinations or variations of learning-based and non-learning-based systems are contemplated.

Further, it has been observed that systems that include sensitive user data, such as health related data, may perform operations on-device so that the user data does not leave the device and thereby become more susceptible to breaches. As such, jaw health system 100 may be located on headphone devices 110A, 110B and may be performed on-device. In addition, it has also been observed that some devices may be restricted with respect to storage, memory, compute resources, and power consumption limitations so that such devices may not be suitable for executing the operations of such systems. As such, aspects of jaw health system 100 may be located in whole or in part on headphones 110A, 110B and companion device 120 so that the multiple subsystems (e.g., imbalance subsystem 160, toughness subsystem 170, etc.) or modules (e.g., bite detection module 162, chew frequency module 164, toughness module 172, optional filters 180A and 180B, etc.) of jaw health system 100 may be located on multiple different devices that may be communicatively linked. In reference to the example of FIG. 2A, optional filters 180A, 180B may be located on headphone devices 110A, 110B, respectively, and imbalance subsystem 160 and toughness subsystem 170 may be located on companion device 120, where imbalance subsystem 160 includes bite detection module 162 and chew frequency module 164, and toughness subsystem 170 includes toughness module 172. In this way, multiple devices may jointly perform the operations of jaw health system 100 so as to overcome any device restrictions with respect to storage, memory, compute resources, power consumption limitations, etc.

In further reference to FIG.2A, jaw health system 100 may include a denoiser 190, where denoiser 190 may include a device or process (e.g., filter, etc.) to remove noise from the sensed inputs of sensors 150 and segregate each bite and/or chew into a left side or right side classification. In addition, it has been observed that the dominant signals related to the ingestion process (e.g., biting, chewing, etc.) may be considered aggressors to concurrent systems (e.g., vital sign systems, etc.) that receive concurrent physiological signals from sensors 150 (e.g., pulse waveform, breathing sounds, etc.) since such dominate signals related to the ingestion process may obscure or interfere with the relatively subtly detected concurrent physiological signals related to the concurrent systems. In such instances, conventional approaches may typically remove such dominant signals detected during the ingestion process to better detect the relatively subtly detected concurrent physiological signals and derive more accurate measurements (e.g., heart rate, respiratory rate, etc.) for each concurrent system. However, in the aspects described, jaw health system 100 may include denoiser 190 that may be configured to separate these overlapping aggressors so that the dominant signals detected during chewing or biting may be extracted to obtain a "clean" bite signal for both headphone devices 110A, 110B, while also allowing each concurrent system (e.g., vital sign systems, etc.) to extract the concurrent physiological signals generated by sensors 150 needed to for their respective measurements (e.g., heart rate, respiratory rate, etc.). In such instances, the operations of denoiser 190 may be performed by non-learning-based systems as well as learning-based systems, where such learning-based systems may include a multiple-input multiple-out ("MIMO") machine learning-based denoiser trained to extract clean bite signals for both headphone devices 110A, 110B.

In aspects, the removal of noise from the sensed inputs of sensors 150 by denoiser 190 may occur before or after jaw health system 100 performs bite/chew determinations. In one aspect, denoiser 190 may be utilized to remove noise from the sensed input of sensors 150 before jaw health system 100 performs any bite/chew determinations. In such instances, the denoising may occur on all of the sensed inputs of sensors 150 (including non-bite/chew signals). In another aspect, denoiser 190 may be utilized to remove noise from the sensed input of sensors 150 after jaw health system 100 performs at least some bite/chew determinations. For example, jaw health system 100 may perform preliminary first pass bite/chew determinations on headphone devices 110A, 110B, where such preliminary bite/chew determinations may be performed by non-learning-based systems (e.g., digital signal processing ("DSP") algorithms, etc.), learning-based systems (e.g., machine learning models, etc.) or any combination thereof in which, for example, a DSP algorithm may pre-process a sensed input signal from sensors 150. In the example of FIG. 2A, jaw health system 100 may include optional filters 180A, 180B located on headphone devices, 110A, 110B, respectively, and may only send signals determined to be bite/chew signals to denoiser 190 so that noise may be removed from such signals. Further, jaw health system 100 may also include a buffer, such as buffer 195 in FIG. 2A, to temporarily store the user data generated by sensors 150 of headphone devices 110A, 110B from denoiser 190, which may include user data from multiple bites. Buffer 195 may then transmit such user data to any of the subsystems or modules of jaw health system 100.

In further reference to FIG. 2A, imbalance subsystem 160 may determine the start/stop indices for each bite. In aspects, imbalance subsystem 160 may utilize non-learning-based systems (e.g., digital signal processing ("DSP") algorithms, etc.), learning-based systems (e.g., machine learning models, etc.), or any combination thereof to determine the start/stop indices for each bite. Further, imbalance subsystem 160 includes bite detection module 162 to determine the start and stop of each bite during the ingestion process. The ingestion process, in which a user ingests or consumes a particular portion of food or other substance, may include at least a first bite in which the user severs a first portion of the food or substance with their teeth, and at least a first chew (and likely periodic chewing) within the first bite to further break down the first portion of the food or substance. The first bite may end when the periodic chewing of the first portion of food subsides for a particular amount of time or when another bite begins, where the ingestion process may include multiple bites and multiple chews within each of the multiple bites, respectively. For example, a first bite and a first chew within the first bite may include severing a first portion of food by the user with their teeth then breaking down the first portion of food by the user with their teeth, where there can be a second chew, a third chew, etc. within the first bite. In reference to FIG. 2A, buffer 195 may transmit signals related to a first bite, a second bite, and a third bite for headphone devices 110A, 110B. In one example, the signals for headphone devices 110A, 110B may be converted into spectrograms, with a first spectrogram related to the first bite, a second spectrogram related to second bite, a third spectrogram related to third bite, etc., where each spectrogram may include start/stop indices to indicate the start and stop of each bite.

In addition, imbalance subsystem 160 may also determine the number of chews within each bite where, for example, a first number of chews within a first bite may be based on first-side user data (e.g., left side) generated by a first set of sensors in a first headphone device (e.g., left headphone) and a second number of chews within the first bite may be based on second-side user data (e.g., right side) generated by a second set of sensors in a second headphone device (e.g., right headphone). In aspects, imbalance subsystem 160 may utilize non-learning-based systems (e.g., digital signal processing ("DSP") algorithms, etc.), learning-based systems (e.g., machine learning models, etc.) or any combination thereof to determine the number of chews within a given bite. In FIG. 2A, imbalance subsystem 160 includes chew frequency module 164 to determine the number of chews within each bite, where chew frequency module 164 may analyze user data (e.g., spectrogram data, etc.) for each bite and determine the number of chew signatures within each bite for each headphone device. For example, for a first bite, headphone device 110A may detect 6 chew signatures, whereas headphone device 110B may detect 9 chew signatures. For a second bite, headphone device 110A may detect 5 chew signatures, whereas headphone device 110B may also detect 5 chew signatures. For a third bite, headphone device 110A may detect 3 chew signatures, whereas headphone device 110B may detect 9 chew signatures.

Based on the user data analyzed by bite detection module 162 and chew frequency module 164, imbalance subsystem 160 may determine whether biting and/or chewing may be skewed towards one side of the mouth or the other within each bite as well as cumulatively over the course of multiple bites by comparing the number of bites detected by headphone devices 110A, 110B. In such instances, imbalance subsystem 160 may determine an imbalance metric, where the imbalance metric may be a continuous number between -1 to +1. For example, metric -1 may be indicative of an excessive biting or chewing imbalance detected by headphone device 110A (e.g., left earbud), metric +1 may be indicative of an excessive biting or chewing imbalance detected by headphone device 110B (e.g., right earbud), and metric 0 may be indicative of no imbalance.

In some aspects, imbalance subsystem 160 may determine the imbalance metric on a per bite basis. In such instances, for the first bite described above, headphone device 110A detected 6 chew signatures and headphone device 110B detected 9 chew signatures. As such, imbalance subsystem 160 may generate an imbalance metric of +1 to indicate excessive biting/chewing detected by headphone device 110B (e.g., right earbud). For the second bite described above, headphone device 110A detected 5 chew signatures and headphone device 110B also detected 5 chew signatures. As such, imbalance subsystem 160 may generate an imbalance metric of 0 to indicate no imbalance. For the third bite described above, headphone device 110A detected 3 chew signatures and headphone device 110B detected 9 chew signatures. As such, imbalance subsystem 160 may generate an imbalance metric of +1 to indicate excessive biting/chewing detected by headphone device 110B (e.g., right earbud).

In other aspects, imbalance subsystem 160 may determine the imbalance cumulatively over discrete timeframes, such as a mealtime (e.g., breakfast, etc.), or extended timeframes (e.g., month, year, etc.). For example, the first, second and third bites described above may be included in such a timeframe. In such instances, for the first, second and third bites described above, headphone device 110A detected 14 chews (6 chew signatures for the first bite, 5 chew signatures for the second bite, and 3 chew signatures for the third bite). Comparatively, for the same first, second and third bites described above, headphone device 110B detected 23 chews (9 chew signatures for the first bite, 5 chew signatures for the second bite, and 9 chew signatures for third bite). As such, for the timeframe spanning the first, second and third bites described above, imbalance subsystem 160 may generate an imbalance metric of +1 to indicate excessive biting/chewing detected by headphone device 110B (e.g., right earbud).

Further, jaw health system 100 may include toughness subsystem 170 to predict a toughness or hardness metric for the food or other substance being chewed based on a toughness threshold. In an aspect, jaw health system 100 may be configured to include a toughness threshold, where foods or substances that result in bite signatures below the toughness threshold may be considered soft, and foods or substances that result in bite signatures above the toughness threshold may be considered hard or tough. In such instances, toughness subsystem 170 may determine a toughness metric that may be represented as either "0" (e.g., soft) or "1" (e.g., hard). In another aspect, toughness subsystem 170 may utilize non-learning-based systems (e.g., digital signal processing ("DSP") algorithms, etc.), learning-based systems (e.g., machine learning models, etc.), or any combination thereof to determine the number of chews within a given bite. In the example of FIG. 2A, toughness subsystem 170 includes toughness module 172 to determine the toughness of a particular food or substance during the ingestion process, where toughness module 172 may analyze user data (e.g., spectrogram data, etc.) for each bite and/or chew to determine whether a toughness signature for each substance exceeds a toughness threshold based on user data generated by the sensors of either headphone. In such instances, toughness subsystem 170 may determine a toughness metric. For example, where a toughness signature is below the toughness threshold as determined by toughness module 172, toughness subsystem 170 may generate a toughness metric of 0 (e.g., soft.). Conversely, where a toughness signature is above the toughness threshold as determined by toughness module 172, toughness subsystem 170 may generate a toughness metric of 1 (e.g., hard).

Jaw health system 100 may generate an overall jaw health metric (e.g., 0-1) based on a linear/non-linear mapping of the imbalance and toughness metrics. For example, the imbalance and toughness metrics may be based on a series of data points indexed or graphed in time order (e.g., time series). As such, the imbalance and toughness metrics may include a distribution of values over the course of multiple different timeframes (e.g., day, week, year, etc.), where the distribution of values may be mapped in order to provide a user side metric. In such instances, jaw health system 100 may map the imbalance metrics, for example, in order to provide the user with user side metrics such as "balanced," "slight imbalance," "moderate imbalance," "severe imbalance," etc. Further, jaw health system 100 may also provide notifications for when a user may be deviating from a goal and/or may cause more severe issues if the mastication or chewing habits remain unaddressed. In some aspects, notifications may be prioritized in that such notifications may occur immediately or more frequently based on a user profile. In one example, where the user has configured a user profile to avoid harder foods (or to prioritize softer foods), jaw health system 100 may prioritize toughness subsystem 170 and accordingly provide a notification when the user consumes a food or substance that exceeds the toughness threshold. In such instances, a processor of jaw health system 100 (e.g., processor 112, processor 122, etc.) may provide an alert or cause a message to be presented on a display of companion device 120 (e.g., "Do not chew hard candy," etc.). In another example, where the user has configured a user profile to include a condition or disorder of the user (e.g., TMJD, etc.), jaw health system 100 may prioritize imbalance subsystem 160 and accordingly provide a notification (or more periodic notifications) when the imbalance metric indicates that the user may further aggravate their condition or disorder without corrective action.

Referring now to FIG. 2B, the jaw health process(es) (e.g., learning-based or non-learning-based systems) of jaw health system 100 may be segmented or compartmentalized to varying degrees. In some aspects, the jaw health processes may be highly segmented or compartmentalized. Referring back to FIG. 2A, the bite/chew determinations as well as the food/substance toughness determination are performed by separate subsystems, namely imbalance subsystem 160 and toughness subsystem 170, respectively. In other aspects, the processes of jaw health system 100 may be collapsed into the same subsystem or module so that rather than multiple smaller subsystems that analyze bite, chew and toughness data separately, a larger singular system may perform such operations together. For example, in reference to the example of FIG. 2B, all such determinations may be performed by jaw health subsystem 200, which may be learning-based or non-learning-based.

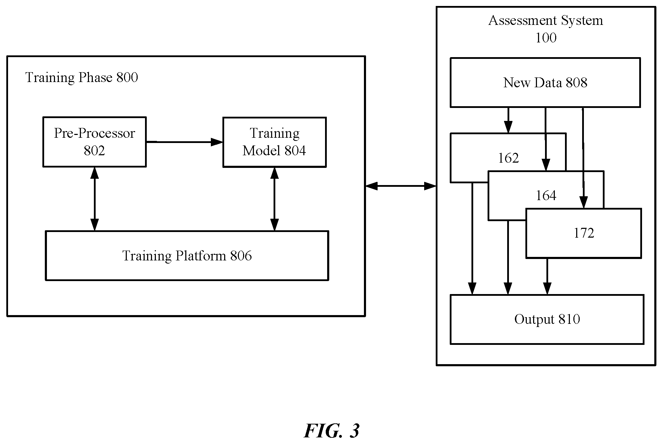

Referring now to FIG. 3, a schematic illustration of a training phase in accordance with an aspect is shown. Whether the imbalance and toughness determinations are segmented into different subsystems such as imbalance subsystem 160 and toughness subsystem 170 as described in FIG. 2A, or collapsed into the same subsystem such as jaw health subsystem 200 as described in FIG. 2B (or any combination thereof), such imbalance and toughness determinations may be governed by learning-based systems (e.g., machine-learning based model, etc.). In such instances, these subsystems can be trained to classify imbalanced chewing (e.g., bite detection, chew frequency, etc.) and/or food toughness using one or more well-known or widely available training techniques such as supervised learning, semi-supervised learning, unsupervised learning, and/or reinforcement learning techniques. For example, in reference to FIG. 3, a diagram of training phase 800 is shown in accordance with aspects. Training phase 800 may include pre-processor 802, training model 804 and training platform 806, where training platform 806 may include a suitable conventional computing device (e.g., one or more GPUs, etc.) and may be implemented as a distributed network of computing devices upon which pre-processor 802 and training model 804 may operate. For example, at pre-processor 802, training data may be provided to illustrate the features that jaw health subsystem 200 seeks to identify and quantify. Such training data may typically include a plurality of training instances, where the training instances may include a vector of values with a corresponding target (e.g., label, value, etc.). Training model 804 may be trained to map from the training data (e.g., an input vector of values) to an estimated target and to ultimately identify and quantify features within new data. Training model 804 may then be deployed to jaw health system 100 and implemented as one of the machine learning models described in accordance with the aspects described. In one example, where the machine learning models of jaw health system 100 may be implemented as segmented models (similar to the example of FIG. 2A), training model 804 may be trained on bite detection data and implemented as bite detection module 162, and additionally trained on chew frequency data and implemented as chew frequency module 164 as part of imbalance subsystem 160. Further, training model 804 may be trained on food toughness data and implemented as toughness module 172 as part of toughness subsystem 170. In another example, where the machine learning models of jaw health system 100 may be implemented as multi-modal models (similar to the examples of FIG. 2B), training model 804 may be trained on a combination of bite detection, chew frequency and/or food toughness data, and may be implemented as bite detection module 162, chew frequency module 164 and toughness module 172 as part of jaw health subsystem 200, for example. Once implemented, the machine learning models of jaw health system 100 may receive new data 808, such as data from the sensors (e.g., sensors 150) of headphone devices 110A, 110B and may then generate a corresponding output 810. It should be noted that training phase 800 may be stored on any combination of the headphone devices, companion devices, and/or on an external computer system (e.g., cloud network, etc.).

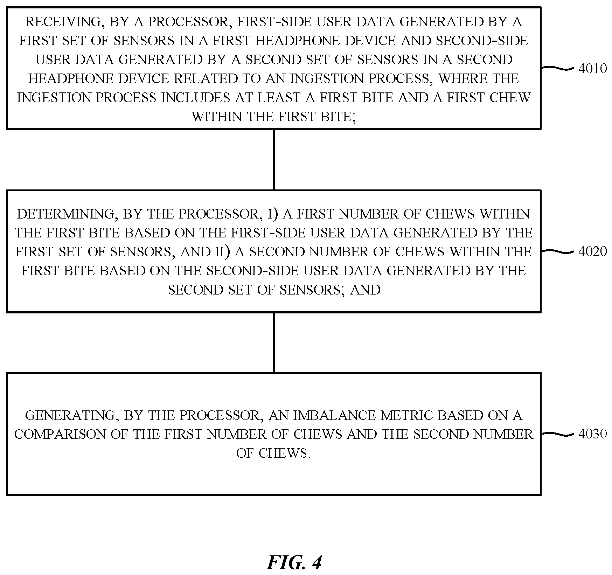

Referring now to FIG. 4, a flow chart of a method for generating a jaw health metric is shown in accordance with an aspect. It should be noted that the operations of jaw health system 100 (e.g., imbalance subsystem 160, toughness subsystem 170, etc.) may be performed by any suitable non-learning based system (e.g., rule-based systems, etc.), learning-based system (e.g., regression techniques, classification techniques, neural networks, and deep learning networks, etc.), or any combination thereof that are well-known or widely available. At operation 4010, sensors 150 of headphone devices 110A, 110B may generate user data (e.g., first-side user data, second-side user data) related to an ingestion process of a particular food or substance (e.g., biting and chewing food), where the ingestion process may include one or more bites and any number of chews within each of the one or more bites. When receiving user data related to the ingestion process generated by sensors 150, a processor (e.g., processor 112, processor 122, etc.) of jaw health system 100 may weight the user data generated by sensors 150 differently based on a mode of operation of the headphone devices, an activity threshold of the user, a playback volume threshold of the headphones, an ambient noise threshold, etc. In addition, based on the user data generated by sensors 150, a processor (e.g., processor 112, processor 122, etc.) of jaw health system 100 may cause optional filters 180A, 180B to perform preliminary bite/chew determinations so that only user data generated by sensors 150 determined to be a bite and/or a chew may be sent "downstream" to be further processed (e.g., denoiser 190, buffer 195, etc.) and analyzed (e.g., imbalance subsystem 160, toughness subsystem 170, etc.). In one example, jaw health system 100 may include denoiser 190 to separate the user data related to the ingestion process (e.g., bites, chews, etc.) from concurrent user data (e.g., pulse rate, etc.) related to concurrent systems (e.g., vital sign system, etc.) so that a processor (e.g. processor 112, processor 122, etc.) of jaw health system 100 may make "clean" bite/chew determinations while each concurrent system may also make its own respective determinations (e.g., heart rate, etc.). In such instances, the signals/data related to the ingestion process as well as the signals/data related to each concurrent system may be generated by sensors 150.

In further reference to FIG. 4, at operation 4020, jaw health system 100 may then analyze the user data to determine the number of bites and the number of chews within each bite over a particular timeframe for each headphone device where, for example, a first headphone device (e.g., first headphone device 110A) may have a first number of chews based on first-side user data generated by the first set of sensors in the first headphone device and the second headphone device (e.g., second headphone device 110B) may have a second number of chews based on second-side user data generated by the second set of sensors in the second headphone device. For example, over a particular timeframe, such as a meal, jaw health system 100 may detect and analyze the number of bites within the meal as well as the number of chews within each bite to determine whether an imbalance is present during the meal. Further, such determinations may be made over longer timeframes (e.g., day, month, year, etc.) to expand the dataset and increase confidence in the imbalance prediction. In addition, jaw health system 100 may also analyze the user data to determine the toughness of a particular food or substance, where the particular food or substance may be "hard" or "soft" based on a toughness threshold set by a user profile. Further, at operation 4030, jaw health system 100 may generate metrics based on the user data generated by sensors 150 of headphone devices 110A, 110B. In one aspect, jaw health system 100 may generate an imbalance metric that may indicate whether the user over-chews on one side of their mouth or the other. In another aspect, jaw health system 100 may generate a toughness metric that may indicate whether the user is biting or chewing a food or substance that exceeds the toughness threshold. In another aspect still, jaw health system 100 may generate an overall jaw health metric that encompasses the imbalance and toughness metrics described above. Further, jaw health system 100 may provide notifications to the user, where such notifications may incorporate the imbalance, toughness and/or overall jaw health metrics. In some instances, the notifications may be prioritized based on a user profile where, for example, the notifications may occur more frequently when a user engages in a behavior (e.g., imbalanced chewing, etc.) that may exacerbate a condition provided in the user profile (e.g., TMJ disorder, etc.), or where the notifications may occur immediately when a user engages in behavior (e.g., eating food that exceeds a toughness threshold, etc.) that contradicts a goal set in the user profile (e.g., eat soft foods, etc.).

In utilizing the various aspects, it would become apparent to one skilled in the art that combinations or variations of the above aspects are possible for the jaw health systems and methods described. Although the aspects have been described in language specific to system features and/or methodological acts, it is to be understood that the appended claims are not necessarily limited to the specific features or acts described. The specific features and acts disclosed are instead to be understood as aspects of the claims useful for illustration.

Some aspects described herein can include the use of learning and/or non-learning-based process(es). The use can include collecting, pre-processing, encoding, labeling, organizing, analyzing, recommending and/or generating data. Entities that collect, share, and/or otherwise utilize user data should provide transparency and/or obtain user consent when collecting such data. The present disclosure recognizes that the use of the data in the jaw health processes can be used to benefit users. For example, the data can be used to train models that can be deployed to improve performance, accuracy, and/or functionality of applications and/or services. Accordingly, the use of the data enables the jaw health processes to adapt and/or optimize operations to provide more personalized, efficient, and/or enhanced user experiences. Such adaptation and/or optimization can include tailoring content, recommendations, and/or interactions to individual users, as well as streamlining processes, and/or enabling more intuitive interfaces. Further beneficial uses of the data in the jaw health processes are also contemplated by the present disclosure.

The present disclosure contemplates that, in some aspects, data used by the jaw health processes includes publicly available data. To protect user privacy, data may be anonymized, aggregated, and/or otherwise processed to remove or to the degree possible limit any individual identification. As discussed herein, entities that collect, share, and/or otherwise utilize such data should obtain user consent prior to and/or provide transparency when collecting such data. Furthermore, the present disclosure contemplates that the entities responsible for the use of data, including, but not limited to data used in association with the jaw health processes, should attempt to comply with well-established privacy policies and/or privacy practices.

For example, such entities may implement and consistently follow policies and practices recognized as meeting or exceeding industry standards and regulatory requirements for developing and/or training the jaw health processes. In doing so, attempts should be made to ensure all intellectual property rights and privacy considerations are maintained. Training should include practices safeguarding training data, such as personal information, through sufficient protections against misuse or exploitation. Such policies and practices should cover all stages of the jaw health processes development, training, and use, including data collection, data preparation, model training, model evaluation, model deployment, and ongoing monitoring and maintenance. Transparency and accountability should be maintained throughout. Such policies should be easily accessible by users and should be updated as the collection and/or use of data changes. User data should be collected for legitimate and reasonable uses of the entity and not shared or sold outside of those legitimate uses. Further, such collection and sharing should occur through transparency with users and/or after receiving the informed consent of the users. Additionally, such entities should consider taking any needed steps for safeguarding and securing access to such data and ensuring that others with access to the data adhere to their privacy policies and procedures. Further, such entities should subject themselves to evaluation by third parties to certify, as appropriate for transparency purposes, their adherence to widely accepted privacy policies and practices. In addition, policies and/or practices should be adapted to the particular type of data being collected and/or accessed and tailored to a specific use case and applicable laws and standards, including jurisdiction-specific considerations.

In some aspects, the jaw health processes may utilize models that may be trained (e.g., supervised learning or unsupervised learning) using various training data, including data collected using a user device. Such use of user-collected data may be limited to operations on the user device. For example, the training of the model can be done locally on the user device so no part of the data is sent to another device. In other implementations, the training of the model can be performed using one or more other devices (e.g., server(s)) in addition to the user device but done in a privacy preserving manner, e.g., via multi-party computation as may be done cryptographically by secret sharing data or other means so that the user data is not leaked to the other devices.

In some aspects, the trained model can be centrally stored on the user device or stored on multiple devices, e.g., as in federated learning. Such decentralized storage can similarly be done in a privacy preserving manner, e.g., via cryptographic operations where each piece of data is broken into shards such that no device alone (i.e., only collectively with another device(s)) or only the user device can reassemble or use the data. In this manner, a pattern of behavior of the user or the device may not be leaked, while taking advantage of increased computational resources of the other devices to train and execute the ML model. Accordingly, user-collected data can be protected. In some implementations, data from multiple devices can be combined in a privacy-preserving manner to train an ML model.

In some aspects, the present disclosure contemplates that data used for the jaw health processes may be kept strictly separated from platforms where the jaw health processes are deployed and/or used to interact with users and/or process data. In such aspects, data used for offline training of the jaw health processes may be maintained in secured datastores with restricted access and/or not be retained beyond the duration necessary for training purposes. In some aspects, the jaw health processes may utilize a local memory cache to store data temporarily during a user session. The local memory cache may be used to improve performance of the jaw health processes. However, to protect user privacy, data stored in the local memory cache may be erased after the user session is completed. Any temporary caches of data used for online learning or inference may be promptly erased after processing. All data collection, transfer, and/or storage should use industry-standard encryption and/or secure communication.

In some aspects, as noted above, techniques such as federated learning, differential privacy, secure hardware components, homomorphic encryption, and/or multi-party computation among other techniques may be utilized to further protect personal information data during training and/or use of the jaw health processes. The jaw health processes should be monitored for changes in underlying data distribution such as concept drift or data skew that can degrade performance of the jaw health processes over time.

In some aspects, the jaw health processes are trained using a combination of offline and online training. Offline training can use curated datasets to establish baseline model performance, while online training can allow the jaw health processes to continually adapt and/or improve. The present disclosure recognizes the importance of maintaining strict data governance practices throughout this process to ensure user privacy is protected.

In some aspects, the jaw health processes may be designed with safeguards to maintain adherence to originally intended purposes, even as the jaw health processes adapt based on new data. Any significant changes in data collection and/or applications of the jaw health process use may (and in some cases should) be transparently communicated to affected stakeholders and/or include obtaining user consent with respect to changes in how user data is collected and/or utilized.

Despite the foregoing, the present disclosure also contemplates aspects in which users selectively restrict and/or block the use of and/or access to data. That is, the present disclosure contemplates that hardware and/or software elements can be provided to prevent or block access to data. For example, in the case of some services, the present technology should be configured to allow users to select to "opt in" or "opt out" of participation in the collection of data during registration for services or anytime thereafter. In another example, the present technology should be configured to allow users to select not to provide certain data for training the jaw health processes and/or for use as input during the inference stage of such systems. In yet another example, the present technology should be configured to allow users to be able to select to limit the length of time data is maintained or entirely prohibit the use of their data for use by the jaw health processes. In addition to providing "opt in" and "opt out" options, the present disclosure contemplates providing notifications relating to the access or use of personal information. For instance, a user can be notified when their data is being input into the jaw health processes for training or inference purposes, and/or reminded when the jaw health processes generate outputs or make decisions based on their data.

The present disclosure recognizes jaw health processes should incorporate explicit restrictions and/or oversight to mitigate against risks that may be present even when such systems having been designed, developed, and/or operated according to industry best practices and standards. For example, outputs may be produced that could be considered erroneous, harmful, offensive, and/or biased; such outputs may not necessarily reflect the opinions or positions of the entities developing or deploying these systems. Furthermore, in some cases, references to or failures to cite third-party products and/or services in the outputs should not be construed as endorsements or affiliations by the entities providing the jaw health processes. Generated content can be filtered for potentially inappropriate or dangerous material prior to being presented to users, while human oversight and/or ability to override or correct erroneous or undesirable outputs can be maintained as a failsafe.

The present disclosure further contemplates that users of the jaw health processes should refrain from using the services in any manner that infringes upon, misappropriates, or violates the rights of any party. Furthermore, the jaw health processes should not be used for any unlawful or illegal activity, nor to develop any application or use case that would commit or facilitate the commission of a crime, or other tortious, unlawful, or illegal act including misinformation, disinformation, misrepresentations (e.g., deepfakes), deception, impersonation, and propaganda. The jaw health processes should not violate, misappropriate, or infringe any copyrights, trademarks, rights of privacy and publicity, trade secrets, patents, or other proprietary or legal rights of any party, and appropriately attribute content as required. Further, the jaw health processes should not interfere with any security, digital signing, digital rights management, content protection, verification, or authentication mechanisms. The jaw health processes should not misrepresent machine-generated outputs as being human-generated.

As described, one aspect of the present technology is the gathering and use of data available from specific and legitimate sources to provide frequent and accurate jaw health metrics. It is contemplated that, in some instances, this gathered data may include personal information data that uniquely identifies or can be used to identify a specific person. Such personal information data can include demographic data, location-based data, online identifiers, telephone numbers, email addresses, home addresses, data or records relating to a user's health or level of fitness (e.g., vital signs measurements, medication information, exercise information, etc.), date of birth, or any other personal information. It is recognized that the use of such personal information data, in the present technology, can be used to the benefit of users. In some instances, health and fitness data may be used, in accordance with the user's preferences to provide insights into their general wellness or may be used as positive feedback to individuals using technology to pursue wellness goals based on jaw health, for example.

It is contemplated that those entities responsible for the collection, analysis, disclosure, transfer, storage, or other use of such personal information data will comply with well-established privacy policies and/or privacy practices. In particular, such entities would be expected to implement and consistently apply privacy practices that are generally recognized as meeting or exceeding industry or governmental requirements for maintaining the privacy of users. Such information regarding the use of personal data should be prominent and easily accessible by users and should be updated as the collection and/or use of data changes. Personal information from users should be collected for legitimate uses only. Further, such collection/sharing should occur only after receiving the consent of the users or other legitimate basis specified in applicable law. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and adapted to applicable laws and standards, including jurisdiction-specific considerations that may serve to impose a higher standard. For instance, in the U.S., collection of or access to certain health data may be governed by federal and/or state laws, such as the Health Insurance Portability and Accountability Act (HIPAA); whereas health data in other countries may be subject to other regulations and policies and should be handled accordingly.

Despite the foregoing, it is also contemplated that aspects in which users selectively block the use of, or access to, personal information data. That is, the present disclosure contemplates that hardware and/or software elements can be provided to prevent or block access to such personal information data. For example, such as in the case with health and fitness data, the present technology can be configured to allow users to select to "opt in" or "opt out" of participation in the collection of personal information data during registration for services or anytime thereafter. In addition to providing "opt in" and "opt out" options, it is contemplated that providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing identifiers, controlling the amount or specificity of data stored (e.g., collecting location data at city level rather than at an address level), controlling how data is stored (e.g., aggregating data across users), and/or other methods such as differential privacy.

Therefore, although the present disclosure broadly covers use of personal information data to implement one or more various disclosed aspects, the present disclosure also contemplates that the various aspects can also be implemented without the need for accessing such personal information data. That is, the various aspects of the present technology are not rendered inoperable due to the lack of all or a portion of such personal information data. For example, content can be selected and delivered to users based on aggregated non-personal information data or a bare minimum amount of personal information, such as the content being handled only on the user's device or other non-personal information available to the content delivery services.

### Numbered Statements of Invention

1. A method for generating jaw health metrics comprising:
   receiving, by a processor, first-side user data generated by a first set of sensors in a first headphone device and second-side user data generated by a second set of sensors in a second headphone device related to an ingestion process, wherein the ingestion process includes at least a first bite and a first chew within the first bite;
   determining, by the processor, i) a first number of chews within the first bite based on the first-side user data generated by the first set of sensors, and ii) a second number of chews within the first bite based on the second-side user data generated by the second set of sensors; and
   generating, by the processor, an imbalance metric based on a comparison of the first number of chews and the second number of chews.
2. The method of statement 1, wherein the first set of sensors and the second set of sensors each include, respectively, at least one of: an external microphone, an error microphone, a voice accelerometer, a motion accelerometer, a gyroscope, and a magnetometer.
3. The method of statement 1, further comprising weighting, by the processor, the first-side user data generated by the first set of sensors and the second-side user data generated by the second set of sensors based on an activity threshold of the user.
4. The method of statement 1, further comprising weighting, by the processor, the first-side user data generated by the first set of sensors and the second-side user data generated by the second set of sensors based on a mode of operation of the first headphone device and the second headphone device.
5. The method of statement 1, further comprising weighting, by the processor, the first-side user data generated by the first set of sensors and the second-side user data generated by the second set of sensors based on a playback volume threshold of the first headphone device and the second headphone device.
6. The method of statement 1, further comprising weighting, by the processor, the first-side user data generated by the first set of sensors and the second-side user data generated by the second set of sensors based on an ambient noise threshold of the first headphone device and the second headphone device.
7. The method of statement 1, further comprising sending, by the processor, the first-side user data generated by the first set of sensors and the second-side user data generated by the second set of sensors to a companion device, wherein analyzing the first-side user data and the second-side user data occurs, at least in part, on the companion device, the companion device being communicatively linked to the first headphone device and the second headphone device.
8. The method of statement 1, further comprising separating, by the processor, the first-side user data and the second-side user data related to the ingestion process from concurrent user data related to concurrent systems, the first-side user data and the second-side user data related to the ingestion process and the concurrent user data related to the concurrent systems being generated by the first set of sensors and the second set of sensors of the first headphone device and the second headphone device, respectively.
9. The method of statement 1, further comprising:
   analyzing, by the processor, the first-side user data and the second-side user data to determine a toughness of a substance chewed during the ingestion process;
   generating, by the processor, a toughness metric for the substance based on a toughness threshold; and
   providing, by the processor, a notification to the user based on the toughness metric, wherein a frequency of the notification occurs based on a user profile.
10. The method of statement 1, further comprising:
   providing, by the processor, a notification to the user based on the imbalance metric, wherein a frequency of the notification occurs based on a user profile.
11. A jaw health system comprising:
   a first headphone device including a first set of sensors; and
   a second headphone device including a second set of sensors;
   wherein the first headphone device and the second headphone device each include a processor and a memory to store instructions that, when executed by the processor, cause the system to perform operations that include:
      receiving, by the processor, first-side user data generated by the first set of sensors in the first headphone device and second-side user data generated by the second set of sensors in the second headphone device related to an ingestion process, wherein the ingestion process includes at least a first bite and a first chew within the first bite;
      determining, by the processor, i) a first number of chews within the first bite detected by the first set of sensors, and ii) a second number of chews within the first bite detected by the second set of sensors; and
      generating, by the processor, an imbalance metric based on a comparison of the first number of chews and the second number of chews.
12. The system of statement 11, wherein the first set of sensors and the second set of sensors each include, respectively, at least one of: an external microphone, an error microphone, a voice accelerometer, a motion accelerometer, a gyroscope, and a magnetometer.
13. The system of statement 11, further comprising weighting, by the processor, the first-side user data generated by the first set of sensors and the second-side user data generated by the second set of sensors based on an activity threshold of the user.
14. The system of statement 11, further comprising weighting, by the processor, the first-side user data generated by the first set of sensors and the second-side user data generated by the second set of sensors based on a mode of operation of the first headphone device and the second headphone device.
15. The system of statement 11, further comprising weighting, by the processor, the first-side user data generated by the first set of sensors and the second-side user data generated by the second set of sensors based on a playback volume threshold of the first headphone device and the second headphone device.
16. The system of statement 11, further comprising weighting, by the processor, the first-side user data generated by the first set of sensors and the second-side user data generated by the second set of sensors based on an ambient noise threshold of the first headphone device and the second headphone device.
17. The system of statement 11, further comprising sending, by the processor, the first-side user data generated by the first set of sensors and the second-side user data generated by the second set of sensors to a companion device, wherein analyzing the first-side user data and the second-side user data occurs, at least in part, on the companion device, the companion device being communicatively linked to the first headphone device and the second headphone device.
18. The system of statement 11, further comprising separating, by the processor, the first-side user data and the second-side user data related to the ingestion process from concurrent user data related to concurrent systems, the first-side user data and the second-side user data related to the ingestion process and the concurrent user data related to the concurrent systems being generated by the first set of sensors and the second set of sensors of the first headphone device and the second headphone device, respectively.
19. The system of statement 11, further comprising:
   analyzing, by the processor, the first-side user data and the second-side user data to determine a toughness of a substance chewed during the ingestion process;
   generating, by the processor, a toughness metric for the substance based on a toughness threshold; and
   providing, by the processor, a notification to the user based on the toughness metric, wherein a frequency of the notification occurs based on a user profile.
20. The system of statement 11, further comprising:
   providing, by the processor, a notification to the user based on the imbalance metric, wherein a frequency of the notification occurs based on a user profile.

## Claims

1. A method for generating jaw health metrics comprising:
receiving, by a processor, first-side user data generated by a first set of sensors in a first headphone device and second-side user data generated by a second set of sensors in a second headphone device related to an ingestion process, wherein the ingestion process includes at least a first bite and a first chew within the first bite;
determining, by the processor, i) a first number of chews within the first bite based on the first-side user data generated by the first set of sensors, and ii) a second number of chews within the first bite based on the second-side user data generated by the second set of sensors; and
generating, by the processor, an imbalance metric based on a comparison of the first number of chews and the second number of chews.

2. The method of claim 1, wherein the first set of sensors and the second set of sensors each include, respectively, at least one of: an external microphone, an error microphone, a voice accelerometer, a motion accelerometer, a gyroscope, and a magnetometer.

3. The method of any preceding claim, further comprising weighting, by the processor, the first-side user data generated by the first set of sensors and the second-side user data generated by the second set of sensors based on an activity threshold of the user.

4. The method of any preceding claim, further comprising weighting, by the processor, the first-side user data generated by the first set of sensors and the second-side user data generated by the second set of sensors based on a mode of operation of the first headphone device and the second headphone device.

5. The method of any preceding claim, further comprising weighting, by the processor, the first-side user data generated by the first set of sensors and the second-side user data generated by the second set of sensors based on a playback volume threshold of the first headphone device and the second headphone device.

6. The method of any preceding claim, further comprising weighting, by the processor, the first-side user data generated by the first set of sensors and the second-side user data generated by the second set of sensors based on an ambient noise threshold of the first headphone device and the second headphone device.

7. The method of any preceding claim, further comprising sending, by the processor, the first-side user data generated by the first set of sensors and the second-side user data generated by the second set of sensors to a companion device, wherein analyzing the first-side user data and the second-side user data occurs, at least in part, on the companion device, the companion device being communicatively linked to the first headphone device and the second headphone device.

8. The method of any preceding claim, further comprising separating, by the processor, the first-side user data and the second-side user data related to the ingestion process from concurrent user data related to concurrent systems, the first-side user data and the second-side user data related to the ingestion process and the concurrent user data related to the concurrent systems being generated by the first set of sensors and the second set of sensors of the first headphone device and the second headphone device, respectively.

9. The method of any preceding claim, further comprising:
analyzing, by the processor, the first-side user data and the second-side user data to determine a toughness of a substance chewed during the ingestion process;
generating, by the processor, a toughness metric for the substance based on a toughness threshold; and
providing, by the processor, a notification to the user based on the toughness metric,
wherein a frequency of the notification occurs based on a user profile.

10. The method of any preceding claim, further comprising:
providing, by the processor, a notification to the user based on the imbalance metric,
wherein a frequency of the notification occurs based on a user profile.

11. A jaw health system comprising:
a first headphone device including a first set of sensors; and
a second headphone device including a second set of sensors;
wherein the first headphone device and the second headphone device each include a processor and a memory to store instructions that, when executed by the processor, cause the system to perform operations that include:
receiving, by the processor, first-side user data generated by the first set of sensors in the first headphone device and second-side user data generated by the second set of sensors in the second headphone device related to an ingestion process, wherein the ingestion process includes at least a first bite and a first chew within the first bite;
determining, by the processor, i) a first number of chews within the first bite detected by the first set of sensors, and ii) a second number of chews within the first bite detected by the second set of sensors; and
generating, by the processor, an imbalance metric based on a comparison of the first number of chews and the second number of chews.

12. The system of claim 11, wherein the first set of sensors and the second set of sensors each include, respectively, at least one of: an external microphone, an error microphone, a voice accelerometer, a motion accelerometer, a gyroscope, and a magnetometer.

13. The system of claim 11 or 12, further comprising weighting, by the processor, the first-side user data generated by the first set of sensors and the second-side user data generated by the second set of sensors based on an activity threshold of the user.

14. The system of any of claims 11 to 13, further comprising weighting, by the processor, the first-side user data generated by the first set of sensors and the second-side user data generated by the second set of sensors based on a mode of operation of the first headphone device and the second headphone device.

15. The system of any of claims 11 to 14, further comprising weighting, by the processor, the first-side user data generated by the first set of sensors and the second-side user data generated by the second set of sensors based on a playback volume threshold of the first headphone device and the second headphone device.
